# EUROPÄISCHE PATENTSCHRIFT

(11) **EP 1 000 354 B1**
(45) Veröffentlichungstag und Bekanntmachung des Hinweises auf die Patenterteilung: **23.10.2002**
(21) Anmeldenummer: 98936247.0
(22) Anmeldetag: 25.06.1998
(51) Int. Cl.: G01N 33/40, F16L 55/162

(54) **SCHLAUCH ZUR ROHRSANIERUNG UND VERFAHREN ZUM MESSEN SEINES AUSHÄRTEGRADS**
FLEXIBLE TUBE FOR REFURBISHING PIPES AND METHOD FOR MEASURING ITS DEGREE OF CURE
TUBE FLEXIBLE POUR RENOVER DES TUYAUX ET PROCEDE POUR MESURER SON DEGRE DE DURCISSEMENT

(30) Priorität: 26.07.1997 DE 19732213; 06.09.1997 DE 19739145
(43) Veröffentlichungstag der Anmeldung: 17.05.2000
(73) Patentinhaber: BKP Berolina Polyester GmbH & Co. KG, 13591 Berlin (DE)
(72) Erfinder: ODENWALD, Ralf, D-16727 Velten (DE)
(74) Vertreter: KOHLER SCHMID + PARTNER
(86) Internationale Anmeldenummer: DE9801732
(87) Internationale Veröffentlichungsnummer: WO99005520

(56) Entgegenhaltungen:
- EP-A- 0 329 277
- US-A- 4 582 520
- US-A- 4 779 452

## Beschreibung

Die Erfindung betrifft in einem ersten Aspekt ein Verfahren zum Messen des Aushärtegrads eines ein Rohr oder dgl. auskleidenden Schlauchs mit aushärtbarer Schlauchwand.

Ein derartiges Messverfahren ist beispielsweise durch die US-A-4 582 520 bekanntgeworden.

Nicht begehbare schadhafte Rohrleitungen werden mit sogenannten Relining-Verfahren saniert, wobei vor Ort aus aushärtbaren Formmassen ein Neurohr erstellt wird. Dazu wird ein mit aushärtbarem Kunstharz getränkter Schlauch in ein zu sanierendes Altrohr eingeführt, positioniert und vor Ort ausgehärtet, wobei als aushärtbares Kunstharz meist wärmeoder lichthärtende Harze verwendet werden. Nachdem der eingeführte Schlauch z.B. mittels Druckluft an die Innenwandung des Altrohrs angepreßt ist, erfolgt entweder eine Warmhärtung durch Füllen des gesamten Schlauches mit Warm-/Heißwasser oder mit Dampf, oder es wird eine Aushärteeinrichtung, z.B. Wärmequellen oder Lampen, mit möglichst gleichbleibender Geschwindigkeit durch den Schlauch gezogen. Unter dem Einfluß der abgegebenen Wärme bzw. des abgestrahlten Lichts härtet dann das Harz aus.

Wenn im Rohr plötzliche Durchmessersprünge, sonstige Unebenheiten etc. auftreten, kann dies zu ruckartigen Änderungen der Geschwindigkeit, mit der sich die Aushärteeinrichtung durch das Rohr bewegt, führen. Dadurch wird der Schlauch an manchen Rohrabschnitten nicht ausreichend ausgehärtet, was die Undichtigkeit des ausgehärteten Neurohrs zur Folge haben kann. Daher ist es wünschenswert, den Aushärtevorgang, insbesondere auch zu reinen Dokumentationszwecken, erfassen, überwachen oder auch überprüfen zu können. Weiterhin muß die Aushärteeinrichtung zum Überwinden von Durchmessersprüngen im Rohr ein entsprechendes Untermaß gegenüber dem Rohr aufweisen, um solche Engstellen passieren zu können. Dadurch befindet sich die Aushärteeinrichtung allerdings nicht immer in der Rohrmitte, so daß der Schlauch auf seinem Umfang nicht gleichmäßig ausgehärtet wird bzw. auf seinem Umfang unterschiedliche Härtungszeiten erforderlich sind.

Aus der eingangs genannten US-A-4 582 520 ist ein Messverfahren bekannt, um den Aushärtungsgrad einer mit einer aushärtbaren Masse beschichteten, im wesentlichen plattenförmigen Bahn zu bestimmen. Dabei wird die Transmission des von einem Sender in Richtung auf die Bahn ausgesandten IR-Lichtes von einem Empfänger gemessen. Das mathematische transformierte Messsignal dient weiterhin zur Steuerung einer Aushärtevorrichtung, im gegenständlichen Fall zur Variation der Temperatur eines Aushärteofens.

Weiterhin ist aus der US-A-4 779 452 ein Viskosimeter zur Ermittlung des Aushärtungsgrades eines plattenförmigen Faserverbundwerkstückes bekannt, bei dem die Viskosität eines Harzes mittels Ultraschall gemessen wird.

Es ist daher die Aufgabe der Erfindung, ein Messverfahren zu schaffen, mit dem der Aushärtegrad eines beliebigen aushärtbaren Gegenstands, insbesondere aber eines in einem zu sanierenden Rohr verlegten Schlauchs, einfach erfasst werden kann.

Diese Aufgabe wird erfindungsgemäß dadurch gelöst, daß in Richtung auf die aushärtbare Schlauchwand Schallwellen, vorzugsweise Ultraschall, oder elektromagnetische Strahlung, vorzugsweise UV-Licht, ausgesandt werden und ihre Reflexion und/oder Transmission durch die aushärtbare Schlauchwand gemessen wird.

Da sich die Transmission von aushärtbaren Materialien bezüglich Schallwellen, wie z.B. Ultraschall, oder bestimmter elektromagnetischer Strahlung, wie z.B. von UV-Licht, entsprechend ihrem Aushärtegrad ändert, kann durch Messen des mit dem Schlauch in Wechselwirkung getretenen reflektierten Schallwellen- bzw. Strahlungsanteils auf den Aushärtegrad des Schlauchs rückgeschlossen werden. Die unterschiedlichen Transmissionsraten ergeben sich insbesondere durch eine veränderte Absorption oder Brechung der Strahlung aufgrund unterschiedlicher Aushärtegrade der Schlauchwand.

Bereits aus den relativen Reflexionsschwankungen auf der axialen Länge des Schlauchs kann auf möglicherweise nicht ausreichend ausgehärtete Abschnitte geschlossen werden. Sind die Transmissions- oder Reflexionsgrade z.B. bezüglich UV-Licht in Abhängigkeit des aushärtbaren Materials, des Schlauchdurchmessers und seiner aushärtbaren Wandstärke bekannt, lassen sich aus den gemessenen Reflexionsgraden auch die zugehörigen absoluten Aushärtegrade ermitteln.

Vorzugsweise werden, wenn der Schlauch in das Rohr eingebracht ist und ausgehärtet wird bzw. ausgehärtet worden ist, Schallwellen bzw. elektromagnetische Strahlung im Innenraum des Schlauchs ausgesandt.

Bei einer ganz besonders bevorzugten Ausgestaltung des erfindungsgemäßen Messverfahrens werden die Schallwellen bzw. wird die elektromagnetische Strahlung außerhalb der aushärtbaren Schlauchwand reflektiert. Die ausgesandte Strahlung tritt dann durch die Schlauchwand hindurch, wird reflektiert und tritt erneut durch die Schlauchwand hindurch und in den Innenraum des Schlauchs ein. Diese Ausführungsform hat den Vorteil, daß die Strahlung die auszuhärtende Schlauchwand zweimal durchdringt und sich dadurch eine Transmissionsänderung im aushärtbaren Material in doppelter Stärke auswirkt. Durch das Durchdringen kann unzureichend ausgehärtetes Material auch in tieferen Schichten nachgewiesen werden.

Um die Aushärtung des Schlauchs auf seiner gesamten Länge erfassen zu können, werden bei bevorzugter Ausgestaltung des Messverfahrens eine die Schallwellen bzw. die elektromagnetische Strahlung abstrahlende Strahlungsquelle und ein reflektierte Schallwellen bzw. Strahlung erfassender Empfänger gemeinsam axial durch den Schlauch bewegt. Dadurch kann die Aushärtung des Schlauchs auf seiner gesamten Länge z.B. dokumentiert werden.

Wenn zumindest der Empfänger reflektierte Schallwellen bzw. elektromagnetische Strahlung im wesentlichen nur rechtwinklig zu seiner Bewegungsrichtung, insbesondere nur in radialer Richtung, empfängt, kann präzise festgestellt werden, an welcher, insbesondere axialen, Stelle der Schlauch nicht ausreichend ausgehärtet ist und gegebenenfalls gezielt eine nachträgliche Aushärtung erfolgen.

Dabei sind bevorzugt Strahlungsquelle und Empfänger auf im wesentlichen gleicher, insbesondere axialer, Höhe angeordnet, damit die Reflexionsstrahlung möglichst nicht durch Streustrahlung beeinträchtigt wird.

Bei einer besonders vorteilhaften Ausgestaltung des erfindungsgemäßen Messverfahrens wird die Reflexion der Schallwellen bzw. der elektromagnetischen Strahlung an unterschiedlichen Umfangsabschnitten des Schlauchs jeweils von einem separaten Empfänger erfasst. So läßt sich feststellen, ob sich unzureichend ausgehärtete Bereiche in einem oberen oder einem unteren Umfangsabschnitt befinden und ob deshalb eine zusätzliche Aushärtung dieses Umfangsabschnittes erforderlich ist. So brauchen z.B. obere Umfangsabschnitte eines waagerecht verlaufenden Wasserrohrs gegebenenfalls nicht einer nachträglichen Aushärtung unterworfen zu werden.

Wenn zusätzlich der Abstand der Strahlungsquelle zur aushärtbaren Wand oder Fläche, insbesondere der Innendurchmesser des Schlauchs, und/oder die Wandstärke der aushärtbaren Schlauchwand gemessen wird, können die gemessenen Transmissions- oder Reflexionswerte in exakte Beziehung zu Durchmesser und Wandstärke des Schlauchs gesetzt werden. Fertigungsoder verlegungstechnisch bedingte Toleranzen der Wandstärke des Schlauchs oder Unebenheiten im zu sanierenden Rohr, die zu Durchmesseränderungen des Schlauchs führen, können so beim Auswerten des Aushärtegrads mitberücksichtigt werden.

Die vorliegende Erfindung betrifft in einem zweiten Aspekt auch einen Schlauch zur Sanierung von Rohren oder dgl. mit einer aushärtbaren Schlauchwand, insbesondere zur Durchführung des oben geschilderten Meßverfahrens.

Bei einem solchen Schlauch ist erfindungsgemäß die aushärtbare Schlauchwand von einer Schallwellen, vorzugsweise Ultraschall, oder elektromagnetische Strahlung, vorzugsweise UV-Licht, reflektierenden Reflexionsschicht umgeben.

Materialien, die mit Hilfe von Licht aushärtbar sind, sind sowohl im nichtausgehärteten, als auch im ausgehärteten Zustand ausreichend lichtdurchlässig, so daß die Transmission der reflektierten Strahlung während des Aushärtevorgangs gemessen und so der Aushärteverlauf verfolgt werden kann.

Bei einer ganz besonders bevorzugten Ausführungsform des erfindungsgemäßen Schlauchs ist die Reflexionsschicht durch eine Folie, vorzugsweise durch einen Folienschlauch, mit reflektierender Innenschicht gebildet. Z.B. kann die reflektierende Innenschicht eine mit einem Metall (z.B. Aluminium) beschichtete Folie sein. Die Wellenlänge der ausgesandten Strahlung ist vorzugsweise auf die Reflexionsschicht entsprechend abgestimmt.

Wenn die Reflexionsschicht bereits eine Schlauchlage des Schlauchs ist, wird ein kostengünstiger, einstückiger Fertigschlauch geschaffen, der vor Ort leicht zu handhaben ist.

Damit das Aushärten zu einem ausreichend meßbaren Effekt führt, werden als aushärtbare Materialien solche gewählt, bei denen sich der Transmissionsgrad, die Absorption oder der Brechungsindex der aushärtbaren Schlauchwand im Wellenlängenbereich der verwendeten Schallwellen bzw. elektromagnetischen Strahlung während des Aushärtens deutlich ändert. So können dem aushärtbaren Material auch Bestandteile, z.B. lichtempfindliche Pigmente, beigefügt werden, die zwar zum Aushärtevorgang selbst nichts beitragen, jedoch dadurch, daß sie der elektromagnetischen Strahlung ausgesetzt werden, sich verfärben und so den Transmissionsgrad verändern. Eine solche Pigmentschicht kann auch als separate Schicht vor der Reflexionsschicht vorgesehen sein.

Weitere Vorteile der Erfindung ergeben sich aus der Beschreibung und der Zeichnung. Ebenso können die vorstehend genannten und die noch weiter aufgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigt:
- Fig. 1: in einem Längsschnitt ein mit einem aushärtbaren Schlauch ausgekleidetes Rohr und einen durch den Schlauch hindurchbewegten Detektor zum Erfassen des Aushärtegrads des Schlauchs;
- Fig. 2: schematisch den Querschnitt des aushärtbaren Schlauchs der Fig. 1; und
- Fig. 3: die Anordnung der Fig. 1, wobei der Detektor zwischen zwei Aushärteeinrichtungen angeordnet ist.

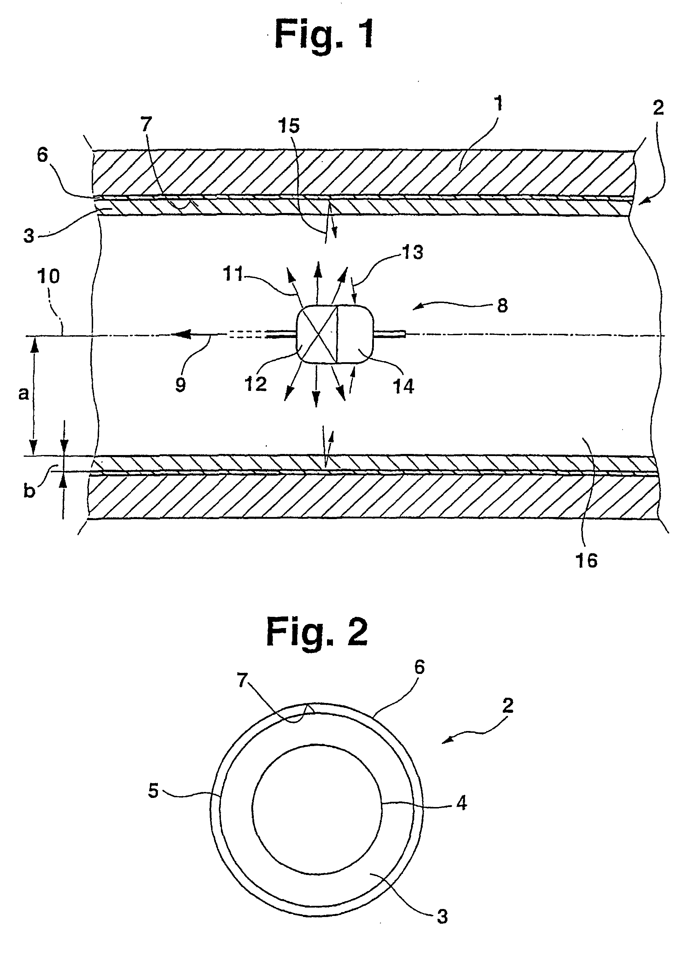

In Fig. 1 ist ein zu sanierendes Rohr **1** gezeigt, das innen mit einem aushärtbaren Schlauch **2** ausgekleidet ist. Dazu weist der Schlauch 2, wie Fig. 2 zeigt, eine aushärtbare Schlauchwand **3** auf, die durch jeweils eine in sich geschlossene Innen- und Außenfolie **4** und **5** begrenzt ist. Zwischen den beiden Folien 4, 5 befindet sich aushärtbares Material, z.B. ein durch Licht oder Wärme aushärtbares Kunstharz. Die Außenfolie 5 ist von einer weiteren äußeren Schlauchlage **6** umgeben, deren Innenseite **7** ultraviolettes(UV)-Licht reflektiert, während die beiden Folien 4, 5 für UV-Licht möglichst durchlässig sind. Bei einer alternativen Ausführungsform kann die Außenfolie 5 auch mit einer entsprechenden reflektierenden Schicht ausgestattet sein.

Nachdem die aushärtbare Schlauchwand 3 durch Wärme- oder Lichteinwirkung ausgehärtet worden ist, wird ein Detektor **8** in axialer Bewegungsrichtung **9** zentriert zur Achse **10** des Schlauchs 2 bzw. des Rohrs 1 durch den Schlauchs 2 hindurchbewegt. Der Detektor 8 umfaßt eine elektromagnetische Strahlung z.B. UV-Licht **11**, abstrahlende Strahlungsquelle **12**, z.B. eine UV-Lampe, und, möglichst auf etwa gleicher axialer Höhe, einen UV-Licht **13** empfangenden Empfänger **14**. Das abgestrahlte UV-Licht 11 durchdringt die Schlauchwand 3, wird an der Innenseite 7 der äußeren Schlauchlage 6 reflektiert, wie durch den Pfeil **15** angedeutet ist, und tritt nach erneutem Durchgang durch die Schlauchwand 3 wieder in den Innenraum **16** des Schlauchs 2 ein. Dort trifft das UV-Licht 13 auf den Empfänger 14, der vorzugsweise nur in nahezu radialer Richtung reflektiertes UV-Licht 13 empfangen kann.

Da sich die Transmission des in der Schlauchwand 3 befindlichen Materials während des Aushärtevorgangs aufgrund z.B. Änderungen im Absorptionsvermögen oder im Brechungsindex ändert, können durch Vergleich des beim Bewegen des Detektors 8 in Bewegungsrichtung 9 mit dem Empfänger 14 gemessenen, reflektierten UV-Lichts 13 relative Unterschiede im Aushärtegrad der Schlauchwand 3 gemessen werden. Dies ermöglicht z.B. eine Dokumentation der Aushärtung des Schlauchs 2 auf seiner gesamten Länge.

Wenn dabei der Detektor 8 durch den Schlauch 2 bzw. das Rohr 1 zentriert zur Achse 10 hindurchgeführt wird, läßt sich aufgrund des Abstands a des Detektors 8 zur Schlauchwand 3 und der Stärke b der Schlauchwand 3 aus dem gemessenen Reflexionsgrad auch der absolute Aushärtegrad aus entsprechenden Tabellen ermitteln. Indem der Empfänger 13 nur nahezu in radialer Richtung reflektiertes UV-Licht 13 empfängt, kann ein gemessener Reflexionswert bzw. Aushärtegrad exakt seinem axialen Entstehungsort im Schlauch zugeordnet werden. Wenn zusätzlich der Innendurchmesser des Schlauchs und auch die Wandstärke der aushärtbaren Schlauchwand 3 gemessen wird, können die gemessenen Transmission- oder Reflexionswerte in exakte Beziehung zu Durchmesser und Wandstärke des Schlauchs gesetzt werden. Fertigungs- oder verlegungstechnisch bedingte Toleranzen der Wandstärke des Schlauchs 2 oder Unebenheiten im zu sanierenden Rohr 1, die zu Durchmesseränderungen des Schlauchs 2 führen, können so beim Auswerten des Aushärtegrads mitberücksichtigt werden.

Der Empfänger 14 kann durch mehrere Einzelempfänger gebildet sein, um zu erfassen, von welchen Umfangsabschnitten des Schlauchs 2 das empfangene Licht 13 reflektiert worden ist. Dies ermöglicht festzustellen, an welchem Umfangsabschnitt, z.B. oben oder unten, sich eine nicht ausreichend ausgehärtete Stelle im Schlauch befindet.

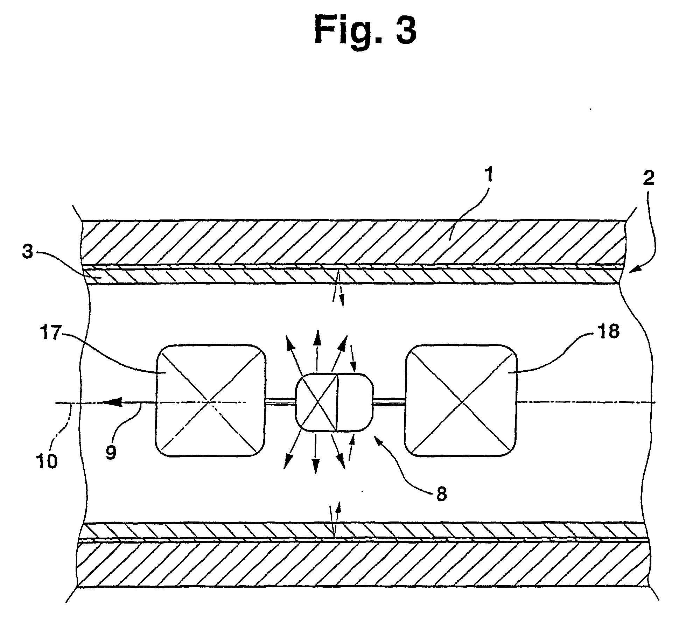

Der Detektor 8 kann auch zur Überwachung und Steuerung des Aushärtevorgangs genutzt werden. Dazu ist der Detektor 8 z.B. in Bewegungsrichtung 9 hinter einer ersten Aushärteeinrichtung **17** und vor einer zweiten Aushärteeinrichtung **18** angeordnet (Fig. 3). Die Wärme- oder Lichtintensität der zweiten Aushärteeinrichtung 18 wird entsprechend dem von dem Detektor 8 ermittelten, durch die erste Aushärteeinrichtung 17 bewirkten Aushärtegrad der Schlauchwand 3 gesteuert. So kann die zweite Aushärteeinrichtung 18 erst bei Erreichen derjenigen Stelle, die von dem Detektor 8 zuvor als von der ersten Aushärteeinrichtung 17 unzureichend ausgehärtet erkannt worden ist, aktiviert und so auch diese Stelle gezielt nachträglich ausgehärtet werden.

Sofern sich das von der Strahlungsquelle 12 ausgesandte UV-Licht 11 ausreichend von der Strahlung unterscheidet, die von einer Aushärteeinrichtung ausgeht, kann der Detektor 8 auch auf gleicher axialer Höhe wie die Aushärteeinrichtung angeordnet werden, wodurch die Geschwindigkeit der Aushärteeinrichtung in Bewegungsrichtung 9 jeweils in Abhängigkeit von dem gerade ermittelten Aushärtegrad der Schlauchwand 3 geregelt werden kann.

Das geschilderte Meßverfahren eignet sich besonders für Schläuche 2, die mit Hilfe von Licht ausgehärtet werden, denn bei diesen Schläuchen 2 sind die Innen- und Außenfolien 4, 5 sowie das aushärtbare Material bereits ausreichend lichtdurchlässig.

Bei einem Verfahren zum Messen des Aushärtegrads eines ein Rohr (1) auskleidenden Schlauchs (2) mit aushärtbarer Schlauchwand (3) wird im Innenraum (16) des Schlauchs (2) elektromagnetische Strahlung (11), insbesondere UV-Licht, ausgesandt und ihre Reflexion und/oder Transmission durch die aushärtbare Schlauchwand (3) gemessen. Vorzugsweise ist dabei die aushärtbare Schlauchwand (3) von einer die elektromagnetische Strahlung (11) reflektierenden Schicht umgeben, die eine Schlauchlage (6) des Schlauchs (2) ist. Durch Messen des mit dem Schlauch in Wechselwirkung getretenen reflektierten Strahlungsanteils kann, wenn sich die Transmission des aushärtbaren Materials bezüglich der elektromagnetischer Strahlung entsprechend ihrem Aushärtegrad ändert, auf den Aushärtegrad des Schlauchs rückgeschlossen werden.

Bei einem Verfahren zum Messen des Aushärtegrads eines Gegenstands mit einer aushärtbaren Wand oder Platte, insbesondere eines ein Rohr (1) auskleidenden Schlauchs (2) mit aushärtbarer Schlauchwand (3), wird in Richtung auf die aushärtbare Wand oder Platte, insbesondere im Innenraum (16, des Schlauchs (2) elektromagnetische Strahlung (11), vorzugsweise UV-Licht, ausgesandt und ihre Reflexion und/oder Transmission durch die aushärtbare Wand oder Platte gemessen. Vorzugsweise ist dabei die aushärtbare Schlauchwand (3) von einer die elektromagnetische Strahlung (11) reflektierenden Schicht umgeben, die eine Schlauchlage (6) des Schlauchs (2) ist. Durch Messen des mit dem Schlauch in Wechselwirkung getretenen reflektierten Strahlungsanteils kann, wenn sich die Transmission des aushärtbaren Materials bezüglich der elektromagnetischen Strahlung entsprechend ihrem Aushärtegrad ändert, auf den Aushärtegrad des Schlauchs rückgeschlossen werden.

## Patentansprüche

1. Verfahren zum Messen des Aushärtegrads eines ein Rohr (1) oder dgl. auskleidenden Schlauchs (2) mit aushärtbarer Schlauchwand (3),
**dadurch gekennzeichnet,**
**daß** in Richtung auf die aushärtbare Schlauchwand (3) Schallwellen, vorzugsweise Ultraschall, oder elektromagnetische Strahlung (11), vorzugsweise UV-Licht, ausgesandt werden und ihre Reflexion und/oder Transmission durch die aushärtbare Schlauchwand (3) gemessen wird.

2. Meßverfahren nach Anspruch 1, **dadurch gekennzeichnet, daß** die Schallwellen bzw. die elektromagnetische Strahlung (11) im Innenraum (16) des Schlauchs (2) ausgesandt wird.

3. Meßverfahren nach Anspruch 1 oder 2, **dadurch gekennzeichnet, daß** die Schallwellen bzw. die elektromagnetische Strahlung (11) außerhalb der aushärtbaren Schlauchwand (3) reflektiert wird.

4. Meßverfahren nach einem der vorhergehenden Ansprüche, **dadurch gekennzeichnet, daß** eine die Schallwellen bzw. die elektromagnetische Strahlung (11) abstrahlende Strahlungsquelle (12) und ein reflektierte Schallwellen bzw. Strahlung (13) erfassender Empfänger (14) gemeinsam axial durch den Schlauch (2) bewegt werden.

5. Meßverfahren nach Anspruch 4, **dadurch gekennzeichnet, daß** zumindest der Empfänger (14) reflektierte Schallwellen bzw. elektromagnetische Strahlung (13) im wesentlichen nur rechtwinklig zu seiner Bewegungsrichtung, insbesondere nur in radialer Richtung, empfängt.

6. Meßverfahren nach Anspruch 4 oder 5, **dadurch gekennzeichnet, daß** Strahlungsquelle (12) und Empfänger (14) in Bewegungsrichtung auf im wesentlichen gleicher, insbesondere axialer, Höhe angeordnet sind.

7. Meßverfahren nach einem der vorhergehenden Ansprüche, **dadurch gekennzeichnet, daß** die Reflexion der Schallwellen bzw. der elektromagnetischen Strahlung (11) an unterschiedlichen Umfangsabschnitten des Schlauchs (2) jeweils von einem separaten Empfänger (14) erfaßt wird.

8. Meßverfahren nach einem der vorhergehenden Ansprüche, **dadurch gekennzeichnet, daß** zusätzlich der Abstand der Strahlungsquelle (12) zur aushärtbaren Schlauchwand (3), insbesondere der Innendurchmesser des Schlauchs (2), und/oder die Wandstärke der aushärtbaren Schlauchwand (3) gemessen wird.

9. Schlauch (2) zur Sanierung von Rohren (1) oder dgl., mit einer aushärtbaren Schlauchwand (3), insbesondere zur Durchführung der Meßverfahrens nach einem der Ansprüche 1 bis 8,
**dadurch gekennzeichnet,**
**daß** die aushärtbare Schlauchwand (3) von einer Schallwellen, vorzugsweise Ultraschall, oder elektromagnetische Strahlung (11), vorzugsweise UV-Licht, reflektierenden Reflexionsschicht umgeben ist.

10. Schlauch nach Anspruch 9, **dadurch gekennzeichnet, daß** die Reflexionsschicht durch eine Folie, vorzugsweise durch einen Folienschlauch, mit reflektierender Innenschicht (7) gebildet ist.

11. Schlauch nach Anspruch 9 oder 10, **dadurch gekennzeichnet, daß** die Reflexionsschicht eine Schlauchlage (6) des Schlauchs (2) ist.

12. Schlauch nach einem der Ansprüche 9 bis 11, **dadurch gekennzeichnet, daß** sich der Transmissionsgrad insbesondere der aushärtbaren Schlauchwand (3) für die Schallwellen bzw. für die elektromagnetische Strahlung (11) während des Aushärtens ändert.

13. Schlauch nach einem der Ansprüche 9 bis 12, **dadurch gekennzeichnet, daß** sich der Brechungsindex der aushärtbaren Schlauchwand (3) für die Schallwellen bzw. für die elektromagnetische Strahlung (11) während des Aushärtens ändert.

## Claims

1. Method for measuring the degree of cure of a tube (2) with curable tube wall (3) lining a pipe (1) or suchlike,
**characterized in that**,
sound waves, preferably ultrasonics, electromagnetic radiation (11), preferably UV-light, are emitted towards the curable tube wall (3) and their reflection and/or transmission through the curable tube wall (3) or plate is measured.

2. Measurement method according to claim 1, **characterized in that**, the sound waves or the electromagnetic radiation (11) are emitted in the interior space (16) of the tube (2).

3. Measurement method according to any of the claims 1 or 2, **characterized in that**, the sound waves or the electromagnetic radiation (11) are reflected outside the curable tube wall (3).

4. Measurement method according to any of the previous claims, **characterized in that**, a radiation source (12), emitting sound waves or electromagnetic radiation (11) and a receiver (14) collecting reflected sound waves or radiation (13) are moved together axially through the tube (2).

5. Measurement method according to claim 4, **characterized in that**, at least the receiver (14) receives reflected sound waves or electromagnetic radiation (13) essentially only orthogonally to its direction of movement, in particular only in radial direction.

6. Measurement method according to any of the claims 4 or 5, **characterized in that**, the radiation source (12) and the receiver (14) are disposed of in direction of movement in essentially the same, particularly axial, position.

7. Measurement method according to any of the previous claims, **characterized in that**, the reflection of the sound waves or electromagnetic radiation (11) of different peripheral sections of the tube (2), is collected each by a separate receiver (14).

8. Measurement method according to any of the previous claims, **characterized in that**, additionally the distance is measured between the radiation source (12) and the curable tube wall (3), in particular the interior diameter of the tube (2), and/or the wall thickness of the curable tube wall (3).

9. Tube (2) for restoring pipes (1) or suchlike with a curable tube wall (3), in particular for the application of the measurement method according to any of the claims 1 through 8,
**characterized in that**,
the curable tube wall (3) is surrounded by a reflection layer reflecting sound waves, preferably ultrasonics, or electromagnetic radiation (11), preferably UV-light.

10. Tube according to claim 9, **characterized in that**, the reflection layer is made of a foil, preferably of a foil tube, with reflecting inner layer (7).

11. Tube according to claim 9 or 10, **characterized in that** the reflection layer is a tube layer (6) of the tube (2).

12. Tube according to any of the claims 9 through 11, **characterized in that**, the transmittance changes, in particular the curable tube wall (3), for the sound waves or for the electromagnetic radiation (11) during curing.

13. Tube according to any of the claims 9 through 12, **characterized in that**, the refractive index changes of the curable wall or plate, in particular of the curable tube wall (3), for the sound waves or for the electromagnetic radiation (11) during curing.

## Revendications

1. Procédé pour mesurer le degré de durcissement d'un tuyau flexible (2) revêtant à l'intérieur un tube (1) ou similaire et comportant une paroi de tuyau durcissable (3), **caractérisé en ce que** l'on émet en direction de la paroi de tuyau durcissable (3) des ondes sonores, de préférence ultrasonores, ou un rayonnement électromagnétique (11), de préférence une lumière ultraviolette, et **en ce que** l'on mesure leur/sa réflexion et/ou transmission à travers la paroi de tuyau durcissable (3).

2. Procédé de mesure selon la revendication 1, **caractérisé en ce que** l'on émet les ondes sonores ou le rayonnement électromagnétique (11) dans le volume intérieur (16) du tuyau (2).

3. Procédé de mesure selon l'une ou l'autre des revendications 1 et 2, **caractérisé en ce que** l'on fait réfléchir les ondes sonores ou le rayonnement électromagnétique (11) à l'extérieur de la paroi de tuyau durcissable (3).

4. Procédé de mesure selon l'une quelconque des revendications précédentes, **caractérisé en ce que** l'on déplace une source de rayonnement (12), émettant les ondes sonores ou le rayonnement électromagnétique (11), conjointement avec un récepteur (14), détectant des ondes sonores réfléchies ou un rayonnement réfléchi (13), axialement à travers le tuyau (2).

5. Procédé de mesure selon la revendication 4, **caractérisé en ce qu'**au moins le récepteur (14) ne reçoit les ondes sonores réfléchies ou le rayonnement électromagnétique réfléchi (13) sensiblement qu'à angle droit par rapport à sa direction de déplacement, en particulier qu'en direction radiale.

6. Procédé de mesure selon l'une ou l'autre des revendications 4 et 5, **caractérisé en ce que** l'on agence la source de rayonnement (12) et le récepteur (14) en direction de déplacement sensiblement à la même hauteur, en particulier axiale.

7. Procédé de mesure selon l'une quelconque des revendications précédentes, **caractérisé en ce que** l'on détecte la réflexion des ondes sonores ou du rayonnement électromagnétique (11) sur des tronçons périphériques différents du tuyau (2) par des récepteurs séparés respectifs (14).

8. Procédé de mesure selon l'une quelconque des revendications précédentes, **caractérisé en ce que** l'on mesure en supplément la distance entre la source de rayonnement (12) et la paroi de tuyau durcissable (3), en particulier le diamètre intérieur du tuyau (2) et/ou l'épaisseur de la paroi de tuyau durcissable (3).

9. Tuyau flexible (2) pour la réfection de tubes (1) ou similaires, comportant une paroi de tuyau durcissable (3), en particulier pour mettre en oeuvre le procédé de mesure selon l'une quelconque des revendications 1 à 8, **caractérisé en ce que** la paroi de tuyau durcissable (3) est entourée par une couche de réflexion réfléchissant des ondes sonores, de préférences ultrasonores, ou un rayonnement électromagnétique (11), de préférence une lumière ultraviolette.

10. Tuyau selon la revendication 9, **caractérisé en ce que** la couche de réflexion est formée par un film, de préférence par un tuyau en film, comportant une couche intérieure réfléchissante (7).

11. Tuyau selon l'une ou l'autre des revendications 9 et 10, **caractérisé en ce que** la couche réfléchissante est une couche (6) appartenant au tuyau (2).

12. Tuyau selon l'une quelconque des revendications 9 à 11, **caractérisé en ce que** le degré de transmission en particulier de la paroi de tuyau durcissable (3) vis-à-vis des ondes sonores ou vis-à-vis du rayonnement électromagnétique (11) se modifie pendant le durcissement.

13. Tuyau selon l'une quelconque des revendications 9 à 12, **caractérisé en ce que** l'indice de réfraction de la paroi de tuyau durcissable (3) vis-à-vis des ondes sonores ou vis-à-vis du rayonnement électromagnétique (11) se modifie pendant le durcissement.
